# EUROPÄISCHE PATENTSCHRIFT

(11) **EP 3 492 876 B1**
(45) Veröffentlichungstag und Bekanntmachung des Hinweises auf die Patenterteilung: **05.01.2022**
(21) Anmeldenummer: 18209526.5
(22) Anmeldetag: 30.11.2018
(51) Int. Cl.: G06Q 10/06, G01D 21/00, G01D 21/02

(54) **SYSTEM UND VERFAHREN ZUM ÜBERWACHEN EINER ARBEITSSITUATION**
SYSTEM AND METHOD FOR MONITORING A WORK SITUATION
SYSTÈME ET PROCÉDÉ DE SURVEILLANCE D'UNE SITUATION DE TRAVAIL

(30) Priorität: 04.12.2017 DE 102017221852
(43) Veröffentlichungstag der Anmeldung: 05.06.2019
(73) Patentinhaber: FRAUNHOFER-GESELLSCHAFT zur Förderung der angewandten Forschung e.V., 80686 München (DE)
(72) Erfinder: Feldhorst, Sascha, 44623 Herne (DE); Grzeszick, René, 44227 Dortmund (DE)
(74) Vertreter: Pfenning, Meinig & Partner mbB

(56) Entgegenhaltungen:
- EP-A1- 2 720 210
- EP-A1- 3 189 310
- EP-A2- 1 971 055
- WO-A1-2017/116802
- US-A1- 2007 159 321
- US-A1- 2016 125 348
- US-A1- 2017 278 052

## Beschreibung

Die Erfindung betrifft ein System zum Überwachen einer Arbeitssituation.

Im industriellen Bereich, beispielsweise in der Fertigung oder in der Logistik, kommen unterschiedliche Überwachungssysteme oder Überwachungsverfahren für Arbeitsprozesse zum Einsatz. Manche Verfahren beruhen beispielsweise auf Videoanalysen, andere auf Prozessbeobachtungen durch einen anwesenden menschlichen Beobachter. Wieder andere erfassen prozesstypische physikalische Größen des Arbeitsprozesses über Beschleunigungssensoren.

Während Methoden, die einen menschlichen Beobachter erfordern, besonders zeitaufwändig und kostenintensiv sind und die Anwesenheit des Beobachters die Messungen außerdem verfälschen kann, haben Methoden, bei denen kein menschlicher Beobachter zugegen ist, den Nachteil, dass eine Umgebung der Arbeit und relevante Kontextinformationen nicht erfasst werden.

Gängige Methoden erfordern weiterhin eine einer Datenerhebung nachgelagerte Datenauswertung, auf deren Basis Prozesse dann zukünftig angepasst werden können.

Ähnliche Systeme sind aus US 2016/125348 A1, US 2017/278052 A1 oder EP 2 720 210 A1 bekannt.

Ziel der Anmeldung ist es, ein System bereitzustellen, welches es ermöglicht, sowohl arbeitsprozessrelevante Informationen als auch Kontextinformationen automatisiert zu erfassen, beide Arten von Informationen miteinander zu verknüpfen und die Daten schnell genug auszuwerten und zu verarbeiten, um Prozessabläufe zum Beispiel im industriellen Bereich, insbesondere in der innerbetrieblichen Logistik, automatisch zu analysieren, zu verbessern und zu steuern. Dabei soll vorzugsweise auch eine vorteilhafte Anpassung von Arbeitsprozessen im laufenden Betrieb ermöglicht werden.

Dies wird durch ein System gemäß Anspruch 1 dieser Anmeldung ermöglicht. Vorteilhafte Ausführungen ergeben sich aus den Unteransprüchen.

Ein solches System umfasst mindestens eine mobile Sensoreinheit, mindestens einen Kommunikationsknoten, mindestens einen durch den Kommunikationsknoten gebildeten oder zusätzlich dazu vorgesehenen Sensorknoten und eine zentrale Auswerteeinheit.

Die mindestens eine mobile Sensoreinheit ist dazu vorgesehen, von einem einen Arbeitsprozess ausführenden Individuum getragen oder benutzt zu werden, und umfasst mindestens einen Sensor zur Messung mindestens einer ersten physikalischen Größe, die zum Beschreiben einer vom jeweiligen Individuum ausgeübten Tätigkeit oder zum Beschreiben eines Zustands des jeweiligen Individuums geeignet ist. Üblicherweise wird dabei für jedes von mehreren Individuen jeweils eine mobile Sensoreinheit bereitgestellt, sodass jeder mobilen Sensoreinheit ein Individuum zugeordnet ist. Erste physikalische Größen im Sinne der vorliegenden Schrift sind insbesondere solche physikalischen Größen, die bei einem Ausführen eines bestimmten Arbeitsprozesses charakteristische Werte annehmen und/oder dazu geeignet sind, eine Ausführung des Arbeitsprozesses zu erkennen, zu analysieren und/oder zu bewerten. Die mindestens eine mobile Sensoreinheit kann also unter anderem zur Erfassung der arbeitsprozessrelevanten Informationen dienen.

Die mindestens eine mobile Sensoreinheit umfasst weiterhin einen Sender zum drahtlosen Übertragen von durch den mindestens einen Sensor dieser mobilen Sensoreinheit erfassten Messwerten der mindestens einen ersten physikalischen Größe an den mindestens einen Kommunikationsknoten.

Weiterhin enthält die mobile Sensoreinheit einen Energiespeicher zur Stromversorgung des Senders. In manchen Ausführungen benötigen auch andere Komponenten der mobilen Sensoreinheit, wie zum Beispiel der Sensor, Strom und können ebenfalls durch den Energiespeicher mit Strom versorgt werden.

Der mindestens eine Sensorknoten umfasst mindestens einen Sensor zur Erfassung mindestens einer zweiten physikalischen Größe, die zum Beschreiben eines Zustands der Umgebung des Sensorknotens geeignet ist. Zweite physikalische Größen im Sinne der Schrift sind also Größen, die einen Zustand der Umgebung des jeweiligen Sensorknotens beschreiben können. Der mindestens eine Sensorknoten kann also unter anderem zur Erfassung von Informationen über eine Arbeitsumgebung oder über einen Arbeitskontext des jeweiligen Individuums, das sich in der Umgebung des jeweiligen Sensorknotens aufhält, dienen. Die ersten physikalischen Größen und die zweiten physikalischen Größen beschreiben dann zusammengenommen die Arbeitssituation des jeweiligen Individuums.

Der mindestens eine Kommunikationsknoten und die mindestens eine mobile Sensoreinheit sind dazu eingerichtet, zumindest in einer Richtung miteinander zu kommunizieren. Dafür weist der mindestens eine Kommunikationsknoten einen Empfänger auf, so dass die von dem mindestens einen Sensor der mindestens einen mobilen Sensoreinheit erfassten ersten physikalischen Größen an den mindestens einen Kommunikationsknoten übermittelt werden können. Bei dem Sender der mobilen Sensoreinheit und dem Empfänger des Kommunikationsknotens kann es sich beispielsweise um einen Bluetooth-Sender und einen Bluetooth-Empfänger handeln.

Der mindestens eine Kommunikationsknoten umfasst weiterhin eine Kommunikationseinheit zum Übertragen der durch den Empfänger dieses Kommunikationsknotens empfangenen Messwerte der mindestens einen ersten physikalischen Größe und von durch den mindestens einen Sensor des Sensorknotens erfassten Messwerten der mindestens einen zweiten physikalischen Größe an die zentrale Auswerteeinheit.

Die zentrale Auswerteeinheit umfasst eine Kommunikationseinheit zum Empfangen der durch die Kommunikationseinheit des Sensorknotens übertragenen Messwerte der mindestens einen ersten physikalischen Größe und der mindestens einen zweiten physikalischen Größe.

Das System ist dazu eingerichtet, die durch den mindestens einen Sensor des Sensorknotens erfassten Messwerte der mindestens einen zweiten physikalischen Größe der zumindest zu einem Zeitpunkt in der Umgebung dieses Sensorknotens lokalisierten mobilen Sensoreinheit zuzuordnen und zusammen mit den durch den mindestens einen Sensor der in der zumindest zu dem Zeitpunkt in der Umgebung dieses Sensorknotens lokalisierten mobilen Sensoreinheit erfassten Messwerten der mindestens einen ersten physikalischen Größe als einander zugeordnet an die zentrale Auswerteeinheit zu übermitteln und dort zu speichern und/oder weiterzuverarbeiten. So sind die zu der Arbeitssituation eines einzelnen Individuums gehörenden ersten und zweiten physikalischen Größen, die zur gleichen Zeit und in der gleichen Umgebung gemessen werden, einander zuordenbar. Es ist somit insbesondere ermittelbar, welche Arbeit wo ausgeführt wird.

Weiterhin kann der Empfänger des mindestens einen Kommunikationsknotens zum Lokalisieren der mindestens einen mobilen Sensoreinheit geeignet sein. Unter Umständen kann es dafür genügen, dass der Empfänger des mindestens einen Sensorknotens die mobile Sensoreinheit oder mindestens eine der mobilen Sensoreinheiten dadurch in einer Umgebung des Sensorknotens lokalisiert, dass eine Signalstärke eines vom Sender der mobilen Sensoreinheit empfangenen Signals eine Schwelle überschreitet. Das Lokalisieren kann aber auch auf andere Weise, zum Beispiel durch eine bidirektionalen Kommunikation, erfolgen, wie später beschrieben wird, wobei in einer Ausführung auch zusätzlich Positionsdaten für die mindestens eine mobile Sensoreinheit ermittelt werden können.

In Ausführungen des beschriebenen Systems wird es vermieden, dass ein Pairing-Prozess stattfinden muss, um die mobile Sensoreinheit mit einem Kommunikationsknoten oder Sensorknoten zu verbinden. Stattdessen kann es beispielsweise vorgesehen sein, dass es etwa durch durchgängiges Senden seitens der mobilen Sensoreinheit und/oder seitens des Sensorknotens ermöglicht wird, Daten immer dann zu übertragen wenn die Sensoreinheit sich in der Nähe eines Kommunikationsknotens bzw. eines Sensorknotens befindet.

Das beschriebene System kann vorteilhaft beispielsweise im industriellen Bereich oder in Krankenhäusern eingesetzt werden. Insbesondere ist es zum Einsatz in Logistik, Produktion oder im Pflegebereich geeignet. Lagermitarbeiter, Produktionsmitarbeiter bzw. medizinisches Personal und/oder Pflegepersonal etc. können dann jeweils mit mobilen Sensoreinheiten ausgestattet werden.

Es kann auch vorgesehen sein, dass ein Individuum mehr als eine mobile Sensoreinheit trägt. Konkret können mobile Sensoreinheiten für ein einziges Individuum so kombiniert werden, dass seine Tätigkeiten möglichst umfassend erfasst und bewertet werden können.

Eine Stromversorgung des mindestens einen Sensorknotens kann mittels Batterie oder Akku oder über ein Stromkabel bereitgestellt werden. Eine bevorzugte Ausgestaltung der Stromversorgung ergibt sich je nach Anwendungsgebiet, beispielsweise abhängig davon, ob in der Nähe des Sensorknotens Steckdosen vorhanden sind und abhängig davon, ob die Komponenten auf dem Sensorknoten, die mit Strom versorgt werden müssen, viel Strom verbrauchen. Da der mindestens eine Sensorknoten üblicherweise nicht häufig bewegt werden muss, kann eine Batterie oder ein Akku des mindestens einen Sensorknotens groß und leistungsfähig sein.

In einer Ausführung kann eine Anonymisierung der Messwerte vorgesehen sein. Eine Zuordnung der gemessenen physikalischen Größen zu einzelnen Individuen, von denen jedes eine mobile Sensoreinheit trägt, wird in dieser Ausführung unmöglich gemacht, beispielsweise indem die Daten, die eine Zuordnung der gemessenen ersten oder zweiten physikalischen Größen zu einer bestimmten mobilen Sensoreinheit ermöglichen würden, verworfen oder gar nicht erhoben werden. Eine Anonymisierung kann zum Schutz der Privatsphäre der Individuen, zum Einhalten rechtlicher Regeln oder um einem Beobachter-Effekt vorzubeugen gewünscht bzw. vorteilhaft sein. Es kann zur Anonymisierung auch vorgesehen sein, dass die Zuordnung der Daten zu den mobilen Sensoreinheiten weiterhin möglich ist, aber Informationen über die Zuordnung der mobilen Sensoreinheit zu dem Individuum nicht erhoben werden oder verworfen werden.

In einer Ausführung, in der mindestens ein Kommunikationsknoten ein Sensorknoten ist, sind dieser mindestens eine Sensorknoten, der auch Sensorknoten ist, und die zentrale Auswerteeinheit dazu eingerichtet, die durch den mindestens einen Sensor des Sensorknotens erfassten Messwerte der mindestens einen zweiten physikalischen Größe der durch den Empfänger dieses Sensorknotens in der Umgebung dieses Sensorknotens lokalisierten mobilen Sensoreinheit zuzuordnen und zusammen mit den durch den mindestens einen Sensor der in der Umgebung dieses Sensorknotens lokalisierten mobilen Sensoreinheit erfassten Messwerten der mindestens einen ersten physikalischen Größe als der mobilen Sensoreinheit zugeordnet zu speichern und/oder weiterzuverarbeiten.

Die mobile Sensoreinheit kann mit einer Batterie bzw. mit einem Akku betrieben werden. Prozesse auf der mobilen Sensoreinheit werden vorzugsweise auf ein Nötigstes reduziert, um Gewicht und Batterie bzw. Akku zu sparen. Die Prozesse, die auf der mobilen Sensoreinheit ausgeführt werden, beschränken sich beispielsweise auf die Messung und ein Übersenden von Messwerten, während andere Schritte, die beispielsweise die Auswertung betreffen, vorzugsweise auf der zentralen Auswerteeinheit ausgeführt werden.

Zur Messung der ersten physikalischen Größen ist der mindestens eine Sensor der mindestens einen mobilen Sensoreinheit als Beschleunigungssensor, Magnetometer oder Gyroskop ausgebildet oder umfasst einen Beschleunigungssensor und/oder ein Magnetometer und/oder ein Gyroskop. Damit lässt sich in einer Ausführung durch ein entsprechendes Tragen oder durch ein entsprechendes Anbringen am Körper des Individuums beispielsweise eine Geschwindigkeit oder eine Beschleunigung von Gliedmaßen beim Ausführen einer bestimmten Bewegung, beispielsweise bei einer Handhabung eines Werkzeugs oder bei einem Ausführen einer Tätigkeit, messen. Diese Geschwindigkeit oder Beschleunigung stellt dann eine erste physikalische Größe dar. Ein anderes Beispiel für eine erste physikalische Größe sind eine Position und eine Haltung von beispielsweise Gliedmaßen oder Wirbelsäule des Individuums. Weiterhin können auch eine am Körper gemessene Temperatur, Vitalwerte, Muskelaktivität oder ein auf ein Körperteil wirkender Druck erste physikalische Größen darstellen, so dass auch Thermometer, ein Pulsmessgerät, Elektroden, beispielsweise für die Elektromyographie (EMG), oder Druck- oder Lagesensoren als Sensoren der mobilen Sensoreinheit möglich sind. Die ersten physikalischen Größen können beispielsweise dazu dienen, eine Tätigkeit zu identifizieren oder dazu, eine korrekte Ausführung der Tätigkeit zu überwachen, beispielsweise um eine gesundheitsschädliche inkorrekte Ausführung zu identifizieren. Die mindestens eine mobile Sensoreinheit kann einen einzigen Sensor oder mehrere verschiedene Sensoren umfassen. In einem System mit mehreren mobilen Sensoreinheiten können die mobilen Sensoreinheiten gleich oder unterschiedlich ausgestaltet sein.

Der mindestens eine Sensor des mindestens einen Sensorknotens ist ein Temperatursensor, ein Helligkeitssensor, ein Vibrationssensor, ein Schallsensor zum Messen einer Umgebungslautstärke, ein Strahlungsnachweisgerät, ein Geigerzähler, ein Dosimeter, ein Barometer, ein Hygrometer oder ein Sensor zur Messung eines Gasgehalts, beispielsweise eines Sauerstoffgehalts, Kohlenstoffmonoxidgehalts oder Kohlenstoffdioxidgehalts, einer Umgebungsluft oder kann einen oder mehrere dieser Sensoren umfassen.

Ein Vorteil des vorgeschlagenen Systems liegt darin, dass eine Ausstattung der mobilen Sensoreinheit auf solche Sensoren beschränkt werden kann, die einen im Wesentlichen direkten Kontakt zu dem die mobile Sensoreinheit tragenden Individuum erfordern, um die Messung von seinen Zustand oder seine Tätigkeit beschreibenden Größen zu ermöglichen. Andere Funktionen können dagegen vorteilhaft auf die Sensorknoten ausgelagert werden. Die mobile Sensoreinheit kann deshalb besonders klein und leicht ausgestaltet sein. Dadurch ermöglicht das vorgeschlagene System, eine Arbeitssituation des jeweiligen Individuums viel vollständiger zu erfassen, als es mit der vergleichsweise klein, leicht und einfach ausgeführten mobilen Sensoreinheit alleine möglich wäre. Ein Nutzen liegt darin, dass das Individuum im Ausführen seiner Tätigkeit nicht beeinträchtigt wird und außerdem vorzugsweise kein psychologischer Beobachtereffekt eintritt. Das kann in manchen Ausführungen weiterhin dadurch unterstützt werden, dass die mobile Sensoreinheit als Wearable, also als am Körper tragbare Einheit, beispielsweise in Form von Kleidung oder ähnlichem, ausgestaltet bzw. in ein solches Wearable integriert wird. Die mobile Sensoreinheit kann beispielsweise als Handschuh oder Armband ausgebildet bzw. in einen Handschuh oder ein Armband integriert sein. Sie kann zum Tragen in einer Tasche geeignet sein oder, beispielsweise zum Überwachen einer Körperhaltung, in die Kleidung integriert sein. Die mobile Sensoreinheit kann auch in ein Werkzeug oder ein Fahrzeug, beispielsweise in einen Gabelstapler, integriert sein. Die Ausgestaltung und Art des Wearables kann beispielsweise von der Art der Sensoren und der zu überwachenden Tätigkeit abhängen. Für manche Anwendungen kann auch ein Mobiltelefon eines Individuums als mobile Einheit verwendet werden.

Für ein einziges Individuum können auch mehrere derartige Wearables kombiniert werden, um seine Bewegungen und Tätigkeiten möglichst umfassend zu umfassen. Die mehreren Wearables des einen Individuums können jeweils eine eigene mobile Sensoreinheit darstellen. Die mehreren Wearables des einen Individuums können aber auch zu derselben mobilen Sensoreinheit gehören und jeweils Sensoren, die zu dieser mobilen Sensoreinheit gehören, aufweisen.

In einer Ausführung kann die mindestens eine mobile Sensoreinheit eine Empfangseinheit aufweisen und der mindestens eine Sensorknoten eine Sendeeinheit. Dadurch kann eine bidirektionale drahtlose Kommunikation zwischen der mindestens einen mobilen Sensoreinheit und dem mindestens einen Sensorknoten ermöglicht werden, um beispielsweise ein Feedback an das die mindestens eine mobile Sensoreinheit tragende Individuum bzw. die Individuen zu senden. Ebenso wird es dadurch ermöglicht, die von dem Sensorknoten erfassten Messwerte an die mobile Sensoreinheit zu übertragen, damit sie von dort an einen Kommunikationsknoten übertragen werden können, falls der Sensorknoten selbst kein Kommunikationsknoten ist. Die bidirektionale drahtlose Kommunikation kann beispielsweise über Bluetooth erfolgen. Die Sendeeinheit des mindestens einen Sensorknotens kann ferner als Funkbake verwendet werden, indem ein Beacon-Signal zur Lokalisierung der mindestens einen mobilen Sensoreinheit gesendet wird. Bei einer Lokalisierung mittels einer Funkbake können auch detaillierte Positionsdaten für die mindestens eine mobile Sensoreinheit erhoben werden und zusammen mit den ersten und zweiten physikalischen Größen und diesen zugeordnet gespeichert und ausgewertet werden.

Die mindestens eine mobile Sensoreinheit kann eingerichtet sein, durch mit ihrer Empfangseinheit empfangene Signale des als Funkbake wirkenden Sensorknotens zu detektieren, wenn sie in der Umgebung dieses Sensorknotens lokalisiert ist.

Bei dem beschriebenen System kann die mobile Sensoreinheit eingerichtet sein, die durch den mindestens einen Sensor des mindestens einen Sensorknotens erfassten Messwerte der mindestens einen zweiten physikalischen Größe mit ihrer Empfangseinheit zu empfangen, wenn sie in der Umgebung dieses Sensorknotens lokalisiert ist. Die mobile Sensoreinheit ist dann weiterhin eingerichtet, diese empfangenen Messwerte zusammen mit den durch ihren mindestens einen Sensor erfassten Messwerten der mindestens einen ersten Physikalischen Größe an den Empfänger des Kommunikationsknotens zu übertragen, wenn oder sobald der Kommunikationsknoten in Reichweite ihres Senders ist.

Außerdem kann die mindestens eine mobile Sensoreinheit einen Speicher aufweisen. Dieser ist eingerichtet zum Zwischenspeichern der durch den mindestens einen Sensor des mindestens einen Sensorknotens erfassten Messwerte der mindestens einen zweiten physikalischen Größe, die die mobile Sensoreinheit mit ihrer Empfangseinheit empfängt. Außerdem ist er eingerichtet, die durch den mindestens einen Sensor der mobilen Sensoreinheit selbst erfassten Messwerte der mindestens einen ersten physikalischen Größe dort zwischenzuspeichern. Das Zwischenspeichern der unterschiedlichen Messwerte erfolgt dabei zeitaufgelöst und zeitlich zugeordnet.

Es kann vorgesehen sein, dass die mobile Sensoreinheit und der Kommunikationsknoten jeweils eine Uhr aufweisen und eingerichtet sind, diese Uhren zu synchronisieren. Dadurch wird es ermöglicht, einen möglichen Drift der Uhr der mobilen Sensoreinheit auszugleichen. Es hat sich herausgestellt, dass ein Drift der Uhr der mobilen Sensoreinheit die Genauigkeit des Systems merklich beeinflussen kann. Die Kommunikationsknoten können beispielsweise über ein Stromnetz mit Energie versorgt werden und genauere Uhren aufweisen als die mobilen Sensoreinheiten. Zeitstempel der im Speicher der mobilen Sensoreinheit zwischengespeicherten Messwerte der ersten und zweiten physikalischen Größen, die dort als zeitaufgelöste einander zugeordnete Messwertpaare vorliegen, können durch einen Abgleich der Uhr der mobilen Sensoreinheit mit der Uhr des Kommunikationsknotens korrigiert werden. Beispielsweise kann die Uhr der mobilen Sensoreinheit zu Beginn der Messung mit der des Kommunikationsknotens synchronisiert werden. Nach Beendigung der Messung, wenn die mobile Sensoreinheit zur Übertragung der auf ihr zwischengespeicherten Messwerte zurück zum Kommunikationsknoten gebracht wird, kann ein möglicher Drift der Uhr der mobilen Sensoreinheit gegenüber der Uhr des Kommunikationsknotens festgestellt und beispielsweise korrigiert werden. Es kann beispielsweise ein lineare Anpassung der Zeitstempel vorgenommen werden.

In einer Ausführung ist die Kommunikationseinheit des mindestens einen Kommunikationsknotens und die komplementäre Kommunikationseinheit der zentralen Auswerteeinheit zur bidirektionalen Kommunikation zwischen dem mindestens einen Kommunikationsknoten und der zentralen Auswerteeinheit ausgebildet sind. Das Feedback kann so von der zentralen Auswerteeinheit über den mindestens einen Kommunikationsknoten an die mindestens eine mobile Sensoreinheit übertragen werden.

Die Kommunikationseinheit des mindestens einen Kommunikationsknotens und die Kommunikationseinheit der zentralen Auswerteeinheit können entweder als drahtlose Kommunikationseinheiten oder als Kommunikationseinheiten, die ein Kabel erfordern, ausgebildet sein. Als Verbindung zwischen den Kommunikationseinheiten kommen alle Verbindungen mit einer ausreichenden Übertragungsrate in Frage. Beispielsweise kann die Übertragung über ein lokales Netzwerk (LAN), ein drahtloses lokales Netzwerk (WLAN), ein Niederenergieweitverkehrnetzwerk (LPWAN), wie beispielsweise NarrowBand loT (NB-loT) oder über ein Mobilfunknetz, beispielsweise 3G, 4G oder 5G erfolgen. Je nach Anwendung und Einsatzort können unterschiedliche Ausführungen bevorzugt sein, beispielsweise abhängig davon, ob eine LAN- oder WLAN-Infrastruktur am Einsatzort zur Verfügung steht.

Zum Senden des Feedbacks kann die mindestens eine mobile Sensoreinheit in einer Ausführung eine Feedback-Vorrichtung zum Übermitteln einer Information an das jeweilige die mobile Sensoreinheit tragende Individuum umfassen. Diese Informationen können beispielsweise Warnungen oder Anweisungen umfassen. Beispielsweise kann die Feedback-Vorrichtung dazu ausgebildet sein, die genannte Information als optisches Signal, als Tonsignal, über eine Displayanzeige, als haptisches Feedback oder als Vibration an das jeweilige die mobile Sensoreinheit tragende Individuum zu senden. So kann das Individuum durch ein Feedback-Signal beispielsweise dazu aufgefordert werden, eine Pause einzulegen, seine Haltung zu korrigieren oder den Raum in dem es sich gerade befindet zu verlassen. Es ist auch möglich, dem Individuum über die Feedback-Funktion eine Aufgabe zuzuweisen, beispielsweise kann das Individuum dazu aufgefordert werden, eine andere Aufgabe an einem anderen Ort zu übernehmen.

Die über die Feedback-Funktion an das Individuum gesendeten Informationen bzw. Anweisungen oder Warnungen können auf den mittels der zentralen Auswerteeinheit verarbeiteten Messwerten der ersten und zweiten physikalischen Größen, bzw. auf den Ergebnissen dieser Auswertung, basieren.

Das Feedback kann aber auch an das Individuum gesendet werden, ohne dass eine Kommunikation mit der zentralen Auswerteeinheit stattgefunden haben muss. Es kann beispielsweise auch basierend auf den von den Sensoren der mobilen Sensoreinheit selbst erfassten Messwerten der ersten physikalischen Größe und/oder basierend auf den an die mobile Sensoreinheit übermittelten Messwerten der zweiten physikalischen Größe gesendet werden.

Es kann vorgesehen sein, dass der mindestens eine Kommunikationsknoten zum Lokalisieren der mobilen Sensoreinheit eingerichtet ist.

In einem System, bei dem der mindestens eine Kommunikationsknoten einen Sensorknoten bildet, können der mindestens eine Kommunikationsknoten und die zentrale Auswerteeinheit eingerichtet sein, die die durch den mindestens einen Sensor des durch diesen Kommunikationsknoten gebildeten Sensorknotens erfassten Messwerte der mindestens einen zweiten physikalischen Größe der durch den Empfänger dieses Kommunikationsknotens in der Umgebung dieses Kommunikationsknotens lokalisierten mobilen Sensoreinheit zuzuordnen und zusammen mit den durch den mindestens einen Sensor der in der Umgebung dieses Kommunikationsknotens lokalisierten mobilen Sensoreinheit erfassten Messwerten der mindestens einen ersten physikalischen Größe als einander zugeordnet zu speichern und/oder weiterzuverarbeiten.

So kann sich ein System zum Überwachen einer Arbeitssituation gemäß dieser Anmeldung ergeben, welches mindestens eine mobile Sensoreinheit, mindestens einen Sensorknoten und eine zentrale Auswerteeinheit umfasst,
wobei die mindestens eine mobile Sensoreinheit mindestens einen Sensor zum Messen mindestens einer ersten physikalischen Größe, die zum Beschreiben einer Tätigkeit oder eines Zustands eines die mobile Sensoreinheit tragenden Individuums geeignet ist, einen Sender zum drahtlosen Übertragen von durch den mindestens einen Sensor dieser mobilen Sensoreinheit erfassten Messwerten der mindestens einen ersten physikalischen Größe an den mindestens einen Sensorknoten und einen Energiespeicher zur Stromversorgung des Senders aufweist,
wobei der mindestens eine Sensorknoten einen Empfänger zum Empfangen der durch den Sender der mobilen Sensoreinheit übertragenen Messwerte der mindestens einen ersten physikalischen Größe und zum Lokalisieren der mobilen Sensoreinheit, mindestens einen Sensor zum Messen mindestens einer zweiten physikalischen Größe, die zum Beschreiben eines Zustands einer Umgebung des Sensorknotens geeignet ist, und eine Kommunikationseinheit zum Übertragen der durch den Empfänger dieses Sensorknotens empfangenen Messwerte der mindestens einen ersten physikalischen Größe und von durch den mindestens einen Sensor dieses Sensorknotens erfassten Messwerten der mindestens einen zweiten physikalischen Größe an die zentrale Auswerteeinheit aufweist und
wobei die zentrale Auswerteeinheit eine Kommunikationseinheit zum Empfangen der durch die Kommunikationseinheit des Sensorknotens übertragenen Messwerte der mindestens einen ersten physikalischen Größe und der mindestens einen zweiten physikalischen Größe aufweist,
wobei der mindestens eine Sensorknoten und die zentrale Auswerteeinheit eingerichtet sind, die durch den mindestens einen Sensor des Sensorknotens erfassten Messwerte der mindestens einen zweiten physikalischen Größe der durch den Empfänger dieses Sensorknotens in der Umgebung dieses Sensorknotens lokalisierten mobilen Sensoreinheit zuzuordnen und zusammen mit den durch den mindestens einen Sensor der in der Umgebung dieses Sensorknotens lokalisierten mobilen Sensoreinheit erfassten Messwerten der mindestens einen ersten physikalischen Größe als einander zugeordnet zu speichern und/oder weiterzuverarbeiten.

Das System kann dazu ausgebildet sein, das Übertragen von Messwerten der mindestens einen ersten physikalischen Größe und gegebenenfalls der Messwerte der mindestens einen zweiten physikalischen Größe von der mindestens einen mobilen Sensoreinheit auf den mindestens einen Kommunikationsknoten und das Übertragen der Messwerte der mindestens einen ersten und der mindestens einen zweiten physikalischen Größen von dem mindestens einen Kommunikationsknoten auf die zentrale Auswerteeinheit sowie eine Verarbeitung der Messwerte der mindestens einen ersten und der mindestens einen zweiten physikalischen Größe mittels der zentralen Auswerteeinheit innerhalb höchstens weniger Sekunden vorzunehmen. Vorzugsweise erfolgen alle genannten Schritte in Echtzeit, also nur mit minimaler, unvermeidbarer Verzögerung. Dafür kommen vorteilhaft modernste Übertragungstechniken sowie Hochleistungsrechner und Hochleistungsalgorithmen zum Einsatz.

Die zentrale Auswerteeinheit kann beispielsweise zur Wiedergabe, zum Auswerten und/oder Speichern von Informationen geeignet sein.

In einer Ausführung werden die Messwerte erster und zweiter physikalischer Größen auf der zentralen Auswerteeinheit mit vorbestimmten Schwellwerten verglichen.

In einer Ausführung werden die Messwerte erster und/oder zweiter physikalischer Größen auf der zentralen Auswerteeinheit mittels Algorithmen des maschinellen Lernens und/oder der Mustererkennung ausgewertet. In einer Ausführung werden heuristische Methoden zur Auswertung der Messwerte verwendet.

In einer Ausführung umfasst oder verwendet die zentrale Auswerteeinheit eine Cloud-Infrastruktur.

In einer Ausführung erfolgt auf der zentralen Auswerteeinheit oder auf der mobilen Sensoreinheit ein ständiger Abgleich der ermittelten ersten und zweiten physikalischen Größen eines Arbeitskontexts jedes Individuums mit Schwellwerten, so dass bei einem Überschreiten eines der Schwellwerte ein entsprechendes Feedback an das jeweilige Individuum im Wesentlichen sofort erfolgen kann.

Die zentrale Auswerteeinheit kann eine Bedienerschnittstelle umfassen. Beispielsweise können Ausgabe- und Eingabegeräte wie Bildschirm und Tastatur bereitgestellt werden, um einem Bediener die Möglichkeit zu geben, das System zu überwachen, die Daten im Blick zu behalten und/oder in das System einzugreifen. Beispielsweise kann für den Bediener eine Möglichkeit bereitgestellt werden, durch ein Eingreifen in das System ein Zuweisen von Aufgaben zu veranlassen oder andere Informationen über die mindestens eine mobile Einheit an das jeweilige die mindestens eine mobile Einheit tragende Individuum zu senden.

Der Aufbau des hier beschriebenen Systems erlaubt einen einfachen Austausch der Sensorknoten bzw. Kommunikationsknoten und der mobilen Sensoreinheiten, beispielsweise zur Wartung oder Erneuerung der Knoten oder Einheiten oder zu einem Laden der Akkus der Knoten oder Einheiten.

In Systemen mit mehreren mobilen Sensoreinheiten und/oder Sensorknoten können die mobilen Sensoreinheiten bzw. Sensorknoten gleich oder verschieden ausgestaltet sein.

In einer Ausführung werden verschiedene Sensorknoten mit unterschiedlichen Sensoren ausgestattet.

In einer Ausführung werden verschiedene mobile Sensoreinheiten mit unterschiedlichen Sensoren und/oder einer Feedback-Funktionen ausgestattet.

In einer Ausführung kann die mindestens eine mobile Sensoreinheit dazu ausgebildet sein, Daten des Sensors der mindestens einen mobilen Sensoreinheit aufzuzeichnen und zu einem späteren Zeitpunkt an einen Kommunikationsknoten zu übermitteln. So können beispielsweise auch Arbeiten oder Bewegungen aufgezeichnet werden, die nicht in der Nähe eines Kommunikationsknotens ausgeführt werden. In einer Ausführung können auch Positionsdaten, beispielsweise Beacon-Signale, die von der mobilen Sensoreinheit empfangen werden, WLAN-Signale von in der Umgebung befindlichen Routern oder GPS-Daten aufgezeichnet und zu einem späteren Zeitpunkt an einen Kommunikationsknoten übermittelt werden. In einer Ausführung werden hierfür neben den Kommunikationsknoten zusätzliche Funkbaken, bei denen es sich nicht um Kommunikationsknoten oder Sensorknoten handelt, bereitgestellt. In einer Ausführung umfasst die mobile Sensoreinheit einen GPS-Empfänger.

In einer Ausführung werden mehr mobile Sensoreinheiten als Sensorknoten bereitgestellt.

Die Anmeldung bezieht sich auch auf Verfahren zur Verwendung des beschriebenen Systems. Alle Merkmale, die im Zusammenhang mit dem System beschrieben wurden, können in derartigen Verfahren verwendet werden.

Ein System gemäß der Anmeldung kann beispielsweise für Prozessanalysen oder zur Prozesssteuerung an Arbeitsstätten, wie beispielsweise in der Produktion, in der Logistik oder in Krankenhäusern eingesetzt werden.

Die Sensorknoten werden an Orten der Arbeitsstätte, insbesondere an neuralgischen Punkten der Arbeitsstätte oder an besonders sicherheitsrelevanten Punkten der Arbeitsstätte bereitgestellt, zum Beispiel in Gassen oder in einer Kommissionierbasis, oder in beispielsweise besonders kalten oder warmen Umgebungen.

Die Kommunikationsknoten können, wenn sie nicht von den Sensorknoten gebildet werden, an Orten der Arbeitsstätte angeordnet werden, an denen die Individuen beispielsweise regelmäßig vorbeikommen. Sie können aber auch beispielsweise in einer Umkleidekabine, an einem Eingang oder in einem Büro bereitgestellt werden.

Ein oder mehrere Individuen, welche an der Arbeitsstätte Tätigkeiten verrichten, können jeweils eine mobile Sensoreinheit tragen.

Ein solches System oder ein solches Verfahren erlaubt eine Verbesserung und Beschleunigung innerbetrieblicher Abläufe, sowie eine Erhöhung der Arbeitssicherheit und eine Verbesserung der Ergonomie.

Beispielhafte Ausführungen sind in den Figuren gezeigt.

Es zeigen, jeweils schematisch,
- Fig. 1: ein System zur Überwachung einer Arbeitssituation mit einer mobilen Sensoreinheit, einem Sensorknoten, der als Kommunikationsknoten ausgebildet ist, und einer zentralen Auswerteeinheit mit ihren jeweiligen Komponenten;
- Fign. 2a-c: unterschiedliche Wearables mit jeweils integrierten mobilen Sensoreinheiten von der Art der mobilen Sensoreinheit aus Fig. 1;
- Fig. 3: ein System zur Überwachung einer Arbeitssituation mit mehreren mobilen Sensoreinheiten, mehreren Sensorknoten, die als Kommunikationsknoten ausgebildet sind, und einer zentralen Auswerteeinheit, bei dem es sich um eine Erweiterung des Systems aus Fig. 1 handelt;
- Fig. 4: eine erste Variation des Systems aus Figur 1;
- Fig. 5: eine zweite Variation des Systems aus Figur 1 und
- Fig. 6: ein System zur Überwachung einer Arbeitssituation mit mehreren mobilen Sensoreinheiten, mehreren Sensorknoten, einem Kommunikationsknoten und einer zentralen Auswerteeinheit, bei dem es sich um eine Erweiterung des Systems aus Fig. 4 oder Fig. 5 handelt.

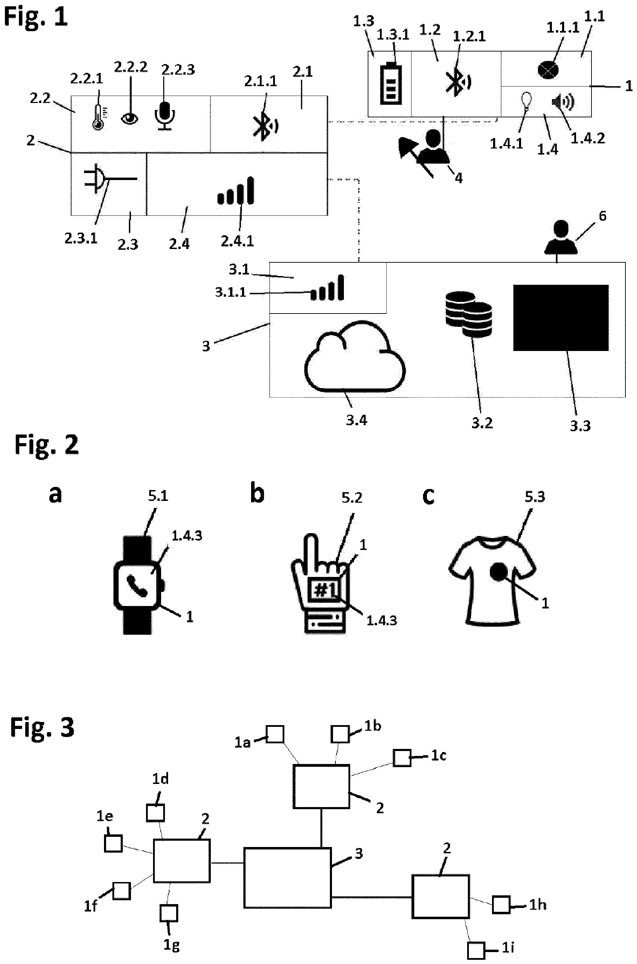

Figur 1 zeigt ein Beispiel für ein erfindungsgemäßes System. Beispielhaft sind eine mobile Sensoreinheit 1, ein Sensorknoten 2 und eine zentrale Auswerteeinheit 3 dargestellt.

Die mobile Sensoreinheit wird bei Verwendung des Systems von einem Individuum 4 beim Verrichten einer manuellen Tätigkeit oder beim Verrichten verschiedener manueller Tätigkeiten bzw. beim Ausführen von Arbeitsprozessen getragen. Die mobile Sensoreinheit 1 weist einen oder mehrere Sensoren 1.1 auf. Der mindestens eine Sensor 1.1 dient zur Erfassung einer oder mehrerer erster physikalischer Größen, die einen Zustand des Individuums 4 während dieser Tätigkeiten oder Prozesse beschreibt bzw. beschreiben. Die mindestens eine erste physikalische Größe dient dazu, zu identifizieren, ob das Individuum 4 gerade eine bestimmte Tätigkeit ausübt oder einen bestimmten Prozess ausführt. Dazu kann der mindestens eine Sensor 1.1 ein Beschleunigungssensor 1.1.1, ein Magnetometer oder ein Gyroskop sein oder einen Beschleunigungssensor und/oder ein Magnetometer und/oder ein Gyroskop umfassen. Die mobile Sensoreinheit weist eine Sendeeinheit oder Sende- und Empfangseinheit 1.2 auf. Die Sendeeinheit oder Sende- und Empfangseinheit 1.2 umfasst eine Bluetooth-Antenne 1.2.1, zusätzlich oder alternativ kann sie aber auch eine andere Antenne umfassen, beispielsweise eine WLAN-Antenne. Vorzugsweise ist die Sendeeinheit oder Sende- und Empfangseinheit 1.2 so ausgebildet, dass sie möglichst wenig Strom verbraucht. Die mobile Sensoreinheit 1 weist eine Energieversorgung 1.3 auf, vorzugsweise eine Batterie oder einen Akku 1.3.1 umfassend. Über die Energieversorgung werden Komponenten der mobilen Sensoreinheit 1, die Strom benötigen, versorgt. Beispielsweise ist die Energieversorgung zur Verwendung einer auswechselbaren Batterie oder eines auswechselbaren Akkus ausgebildet, die bzw. der während einer Verwendung des Systems schnell gewechselt werden kann, so dass die mobile Sensoreinheit jederzeit zur Verfügung steht und nicht zu einem Laden an eine Ladestation angeschlossen werden muss.

Die mobile Sensoreinheit weist weiterhin eine Feedback-Funktion 1.4 auf. Die Feedback-Funktion 1.4 umfasst eine oder mehrere Einheiten zum Senden eines Signals an das Individuum 4. Sie kann beispielsweise ein Feedback-Lämpchen 1.4.1 und/oder einen Feedback-Lautsprecher 1.4.2 umfassen, um einfache Blink- oder Tonsignale an das Individuum zu senden. Es kann aber auch andere Arten von Feedback-Funktionen umfassen, beispielsweise eine Vibrationseinheit zum Senden eines haptischen Feedbacks oder ein Display.

Die mobile Sensoreinheit kann zusätzlich oder alternativ zu den in der Figur 1 gezeigten Komponenten noch weitere Komponenten aufweisen, beispielsweise zusätzliche Sensoren neben dem Beschleunigungssensor 1.1.1. Es können auch Komponenten, beispielsweise die Feedback-Funktion 1.4, weggelassen werden. Je nachdem, welche Arten von Prozessen analysiert werden sollen und ob laufende Prozesse gesteuert werden sollen, ergibt sich die genaue Ausgestaltung der Sensoren 1.1, der Energieversorgung 1.3, der Kommunikationseinheit 1.2 und der Feedback-Funktion 1.4 für den Fachmann.

Der Sensorknoten 2 umfasst eine Empfangseinheit oder Sende- und Empfangseinheit 2.1. Die Empfangseinheit oder Sende- und Empfangseinheit 2.1 ist zur Kommunikation mit der Sendeeinheit oder Sende- und Empfangseinheit 1.2 der mobilen Sensoreinheit 1 ausgebildet und kann dementsprechend ebenso eine Bluetooth-Antenne 2.1.1 und/oder eine andere Antenne umfassen. Die Empfangseinheit oder Sende- und Empfangseinheit 2.1 kann zur Verwendung als Funkbake, zu einer Ortung der mobilen Sensoreinheit 1 oder mehrerer mobiler Einheiten, eingerichtet sein, um Positionsdaten der mobilen Sensoreinheit 1 oder mehrerer mobilen Einheiten zu erfassen. Außerdem ist die Empfangseinheit oder Sende- und Empfangseinheit 2.1 zum Empfangen der von dem Sensor 1.1 erfassten einen oder mehreren ersten physikalische Größen eingerichtet, welche über die Sendeeinheit oder Sende- und Empfangseinheit 1.2 der mobilen Sensoreinheit 1 verschickt werden. Die Kommunikation zwischen der mobilen Sensoreinheit 1 und dem Sensorknoten 2 ist in der Figur durch eine gestrichelte Linie angedeutet.

Der Sensorknoten 2 umfasst einen oder mehrere Sensoren 2.2 zur Messung einer oder mehrerer zweiter physikalischer Größen. Beispielsweise kann der mindestens eine Sensor 2.2 ein Temperatursensor 2.2.1, ein Helligkeitssensor 2.2.2 oder ein Schallsensor 2.2.3 sein oder einen oder mehrere davon umfassen. Unter den Sensoren 2.2 können auch noch weitere Sensoren sein, wie beispielsweise ein Strahlungsnachweisgerät, ein Sensor zur Messung von Vibrationen oder von Luftdruck, oder Sensoren beispielsweise zur Messung eines Sauerstoffgehalts oder Kohlenstoffmonoxidgehalts oder Kohlenstoffdioxidgehalts einer Umgebungsluft. Diese zweiten physikalischen Größen beschreiben ein Umfeld des Sensorknotens, in welchem die manuelle Tätigkeit von dem Individuum 4, welches die mobile Sensoreinheit 1 mit sich führt, die mit dem Sensorknoten 2 in Kommunikation steht, ausgeführt wird. Die zweiten physikalischen Größen müssen dabei nicht direkt an der mobilen Sensoreinheit gemessen werden, da sie beispielsweise in einem ganzen Raum, der das Umfeld, in welchem die manuelle Tätigkeit ausgeführt wird, darstellt, konstant oder ungefähr konstant sind. Das kann beispielsweise bei einer Temperatur, bei einer Helligkeit, einer Strahlenbelastung, einer Gaskonzentration oder einer Lautstärke gegeben sein. Beispielsweise kann das Umfeld ein Kühlraum sein oder eine unmittelbare Umgebung eines Ofens oder einer Strahlungsquelle oder Abgasquelle, so dass niedrige oder hohe Temperaturen oder eine radioaktive Strahlung oder Luftverschmutzung herrschen, die das Individuum schädigen könnten, wenn sich das Individuum dort zu lange aufhält, insbesondere wenn es dabei Tätigkeiten verrichtet. Diese zweiten physikalischen Größen müssen nicht auf der mobilen Sensoreinheit 1 gemessen werden. Zur Analyse der Arbeitssituation des die mobile Sensoreinheit 1 tragenden Individuums reicht es aus, die zweiten physikalischen Größen an dem Sensorknoten zu messen und die Ortung bzw. Lokalisierung der mobilen Sensoreinheit 1 vorzunehmen. Somit können Prozesse, die auf der mobilen Sensoreinheit ausgeführt werden, minimiert werden, bzw. es können Messgeräte bereitgestellt werden, die zu groß sind, um von dem Individuum 4 getragen zu werden oder die zu viel Strom brauchen, um von der Energieversorgung 1.3 der mobilen Sensoreinheit 1 gespeist zu werden. Dadurch werden auch die Kosten der mobilen Sensoreinheit 1 reduziert. Dies ist insbesondere dann bevorzugt, wenn deutlich mehr mobile Sensoreinheiten 1 als Sensorknoten 2 zu dem System gehören.

Das System ist dazu eingerichtet, eine beispielsweise gefährdende Situation für das Individuum am Überschreiten bestimmter Grenzwerte zu erkennen. Wenn beispielsweise, wie oben beschrieben, das Umfeld ein Kühlraum oder eine unmittelbare Umgebung eines Ofens ist, so kann das System an das Individuum 4, welches sich dort aufhält oder dort Tätigkeiten verrichtet, nach einer vorgegebenen Zeit eine Warnung über die Feedback-Funktion 1.4 senden und das Individuum 4 zu einer Unterbrechung der Tätigkeit oder zu einem Verlassen der Umgebung auffordern.

In unterschiedlichen Umgebungen unterschiedlicher Sensorknoten 2 können unterschiedliche Sensoren 2.2 sinnvoll sein. Durch eine Anordnung der Sensoren für die zweiten physikalischen Größen an dem Sensorknoten kann in jedem Umfeld eine Beschränkung auf diejenigen Sensoren erfolgen, die in dem jeweiligen Umfeld benötigt werden. Durch eine Zentralisierung der Messung zweiter physikalischer Größen an den Sensorknoten 2 können ferner Kosten gespart werden. Der Sensorknoten 2 weist eine Energieversorgung 2.3 auf. Die Energieversorgung 2.3 kann einen Netzstecker 2.3.1 umfassen, um den Sensorknoten 2 möglichst wartungsfrei zu halten und kein Austauschen von Batterien oder Akkus erforderlich zu machen. Der Sensorknoten 2 kann aber stattdessen auch mit einer Batterie oder mit einem Akku betrieben werden, um keine zusätzliche Infrastruktur, wie beispielsweise ein Stromnetz, erforderlich zu machen.

Der Sensorknoten 2 ist gleichzeitig als Kommunikationsknoten ausgebildet. Das heißt, er weist weiterhin eine Kommunikationseinheit 2.4 zur Kommunikation mit der zentralen Auswerteeinheit 3 auf. Die Kommunikationseinheit 2.4 ist vorzugsweise als drahtlose Kommunikationseinheit ausgebildet und umfasst beispielsweise eine Antenne 2.4.1 zur Nutzung eines Mobilfunknetzes oder eines anderen drahtlosen Netzwerks. Vorzugsweise werden aktuelle Hochgeschwindigkeitsmobilfunkstandards zur Kommunikation verwendet, beispielsweise 3G oder LTE bzw. 4G oder 5G, oder ein Niedrigenergieweitverkehrnetzwerk (LPWAN), wie beispielsweise NarrowBand loT (NB-loT). Die Kommunikationseinheit 2.4 kann aber auch für WLAN-Kommunikation oder eine Verbindung mit Kabeln ausgestaltet sein.

Die zentrale Auswerteeinheit 3 umfasst eine Kommunikationseinheit 3.1 zur Kommunikation mit der Kommunikationseinheit 2.4 des Sensorknotens 2. Dementsprechend ist die Kommunikationseinheit 3.1 der zentralen Auswerteeinheit 3 wie die Kommunikationseinheit 2.4 des Sensorknotens 2, beispielsweise als drahtlose Kommunikationseinheit, beispielsweise eine Mobilfunkantenne 3.1.1. umfassend, ausgestaltet oder, in einer anderen Ausführung, für eine Verbindung mit Kabeln ausgestaltet. Die Kommunikation zwischen der zentralen Auswerteeinheit 3 und dem Sensorknoten 2 ist in der Figur durch eine gestrichelte Linie angedeutet.

Die zentrale Auswerteeinheit 3 ist zu einem Speichern und Verarbeiten bzw. Auswerten von Daten, die von dem Sensorknoten 2 und von der mindestens einen mobilen Sensoreinheit 1, über den Sensorknoten 2, an die zentrale Auswerteeinheit übermittelt werden, ausgestaltet. Diese Daten umfassen die ersten und zweiten physikalischen Größen und können in manchen Ausführungen auch Positionsdaten umfassen. Hierfür umfasst oder verwendet die zentrale Auswerteeinheit eine Datenbank 3.2, in der Rohdaten erster und zweiter physikalischer Größen, Positionsdaten und Auswertungsergebnisse gespeichert werden können. Die ersten und zweiten physikalischen Größen, werden vorzugsweise zugeordnet gespeichert, so dass erste physikalische Größen, die von einer bestimmten mobilen Sensoreinheit 1 an einen bestimmten Sensorknoten 2 übermittelt werden und von diesem bestimmten Sensorknoten an die zentrale Auswerteeinheit 3 weiterübermittelt werden, den zeitgleich von dem bestimmten Sensorknoten 2 erfassten zweiten physikalischen Größen zugeordnet sind. Die an die zentrale Auswerteeinheit 3 übermittelten Messwerte der ersten physikalischen Größen werden mit Hilfe von Algorithmen des Maschinellen Lernens einer bestimmten Tätigkeit zugeordnet, zur Identifikation eines bestimmten Arbeitsprozesses. Der identifizierte Arbeitsprozess wird mit den zweiten physikalischen Größen verknüpft. Damit ist zu einem bestimmten Zeitpunkt der Messung die Arbeitssituation des diese bestimmte mobile Sensoreinheit tragenden Individuums 4, die einen Zustand des Individuums 4 und einen Zustand des Umfelds des Individuums 4 umfasst, als solche identifizierbar. Ferner ist auch eine Position des Individuums 4 durch die Zuordnung seiner bestimmten mobilen Sensoreinheit 1 zu dem bestimmten Sensorknoten 2 zu dem bestimmten Zeitpunkt der Messung, bzw. in manchen Ausführungen auch durch die parallel erhobenen Positionsdaten, erfasst. Zur Analyse der Prozesse können die Daten mehrerer mobiler Sensoreinheiten statistisch ausgewertet werden. Zur Aus- und Weiterverarbeitung der Daten aus der Datenbank 3.2 kann die zentrale Auswerteeinheit, zur Optimierung der Auswertung der Daten, insbesondere bei großen Datenmengen oder bei hohen und/oder schwankenden benötigten Kapazitäten, Cloud-Technologie 3.4 verwenden.

Die zentrale Auswerteeinheit 3 kann weiterhin eine Bedienerschnittstelle 3.3 umfassen, mittels welcher einem Bediener 6 des Systems Daten angezeigt werden können und/oder mittels welcher der Bediener 6 in das System eingreifen kann. Hierfür kann die Bedienerschnittstelle 3.3 beispielsweise Ausgabegeräte, wie zum Beispiel einen Bildschirm zum Anzeigen einer Benutzeroberfläche und Eingabegeräte, wie zum Beispiel Tastatur und Maus, umfassen.

Figuren 2a-c zeigen unterschiedliche Wearables 5.1, 5.2, 5.3, in die jeweils eine mobile Sensoreinheit 1 integriert ist. Die jeweils integrierte mobile Sensoreinheit 1 ist jeweils beispielsweise wie in Figur 1 gezeigt oder ähnlich wie in Figur 1 gezeigt aufgebaut und kann alle oder einen Teil der Komponenten der in der Figur 1 gezeigten mobilen Sensoreinheit 1 aufweisen. Beispielsweise kann die Feedback-Funktion 1.4 oder eine der möglichen Ausführungen der Feedback-Funktionen 1.4.1, 1.4.2 weggelassen werden oder anders ausgebildet sein. Die mobile Sensoreinheit kann auch zusätzlich oder statt der Komponenten der in der Figur 1 gezeigten mobilen Sensoreinheit 1 weitere, nicht in Figur 1 gezeigte Komponenten aufweisen, beispielsweise zusätzliche Sensoren neben dem Beschleunigungssensor 1.1.1. Je nachdem, welche Art von Prozess analysiert werden soll, ergibt es sich für den Fachmann, in welche Art von Wearable die mobile Sensoreinheit 1 jeweils vorteilhaft integriert wird, welche Sensoren verwendet werden und wie die Sensoren, die Energieversorgung 1.3, die Kommunikationseinheit 1.2 und die Feedback-Funktion 1.4 der mobilen Sensoreinheit 1 vorteilhaft ausgestaltet sind.

Figur 2a zeigt ein Armband 5.1 bzw. ein armbanduhrenähnliches Wearable, in welches die mobile Sensoreinheit 1 eingebaut ist. Die mobile Sensoreinheit 1 umfasst in dem gezeigten Beispiel die Feedback-Funktion 1.4, die ein Feedback-Display 1.4.3 umfasst, über welches dem Individuum 4 Mitteilungen wie beispielsweise Warnungen oder Anweisungen angezeigt werden können. Die in das Armband 5.1 eingebaute mobile Sensoreinheit 1 umfasst beispielsweise weiterhin einen als Beschleunigungssensor 1.1.1 ausgebildeten Sensor 1.1, der dazu geeignet ist, Armbewegungen des Individuums zu detektieren.

Figur 2b zeigt ein als Handschuh 5.2 ausgebildetes Wearable, in welches die mobile Sensoreinheit 1 integriert ist. Die mobile Sensoreinheit 1 weist beispielsweise einen Sensor 1.1 auf, der einen Beschleunigungssensor 1.1.1 und einen Sensor zur Detektion einer Fingerstellung von Handschuhfingern, mit welchem sich beispielsweise feststellen lässt, ob eine Hand des den Handschuh 5.2 tragenden Individuums 4 geöffnet oder geschlossen ist, umfasst. Weiterhin ist in dem gezeigten Beispiel auf einem Handrücken des Handschuhs 5.2 ein Feedback-Display 1.4.3 angeordnet.

Figur 2c zeigt ein als T-Shirt 5.3 ausgebildetes Wearable mit integrierter mobiler Einheit 1. Die mobile Sensoreinheit 1 kann, wenn sie in ein T-Shirt integriert ist, beispielsweise Sensoren 1.4 aufweisen, mit welchen sich eine Position eines Oberkörpers oder eine Wirbelsäulenstellung des Individuums 4 detektieren lässt.

Figur 3 zeigt ein System gemäß der Anmeldung mit mehreren mobilen Sensoreinheiten 1a-i und mehreren Sensorknoten 2, die jeweils als Kommunikationsknoten ausgebildet sind. Jede der mobilen Sensoreinheiten 1a-i und jeder der Sensorknoten 2 ist dabei so oder so ähnlich ausgestaltet wie die mobilen Sensoreinheiten 1 und die Sensorknoten 2 aus den Figuren 1 und 2. Die genaue Ausgestaltung der einzelnen mobilen Sensoreinheiten 1a-i und der einzelnen Sensorknoten 2 kann dabei jeweils gleich oder jeweils verschieden sein. Die Anzahl der mobilen Sensoreinheiten 1a-i und der Sensorknoten 2 ist in der Figur beispielhaft gewählt. Es können beliebig viele Sensorknoten 2 und beliebig viele mobile Sensoreinheiten 1 zu dem System gehören. Die Sensorknoten 2 sind dabei jeweils, wie in der Figur 3 mit Linien angedeutet, als Kommunikationsknoten mit der zentralen Auswerteeinheit 3 in Kommunikation, beispielsweise durch ein Mobilfunknetz, eine 3G, 4G oder 5G-Verbindung, über ein Niedrigenergieweitverkehrnetzwerk (LPWAN), wie beispielsweise NarrowBand loT (NB-loT), oder über ein Lokales Netzwerk, welches drahtlos, zum Beispiel als WLAN, oder mit Kabelverbindungen ausgestaltet sein kann. Die mobilen Sensoreinheiten 1a-i stehen jeweils mit einem der Sensorknoten 2 in Kommunikation, beispielsweise über eine Bluetooth-Verbindung. Jeder der Sensorknoten 2 kann beispielsweise als Funkbake verwendet werden undsendet ein Beacon-Signal, wodurch eine Lokalisierung der in der Nähe des jeweiligen Sensorknotens 2 liegenden mobilen Sensoreinheiten ermöglicht wird. Basierend auf einer Position jeder mobilen Sensoreinheit 1a-i kann jede der mobilen Sensoreinheiten 1a-i einem der Sensorknoten 2 zugeordnet werden und sich mit diesem drahtlos verbinden und beispielsweise eine bidirektionale Datenverbindung herstellen. Wie durch Linien angedeutet, sind die mobilen Sensoreinheiten 1a-c dem Sensorknoten 2b zugeordnet, die mobilen Sensoreinheiten 1d-1g dem Sensorknoten 2c und die mobilen Sensoreinheiten 1i-h dem Sensorknoten 2c. Diese Zuordnung kann beispielsweise erfolgen, weil sich die mobilen Sensoreinheiten 1a-i jeweils im gleichen Raum befinden wie der jeweils zugeordnete Sensorknoten 2 oder weil die mobilen Sensoreinheiten 1a-i höchstens einen bestimmten Abstand zu dem jeweils zugeordneten Sensorknoten 2 aufweisen. Wird eine der mobilen Sensoreinheiten 1a-i von dem jeweils zugeordneten Sensorknoten 2 wegbewegt, hin zu einem anderen der Sensorknoten 2, kann diese eine der mobilen Sensoreinheiten 1a-i dem anderen der Sensorknoten 2 zugeordnet werden.

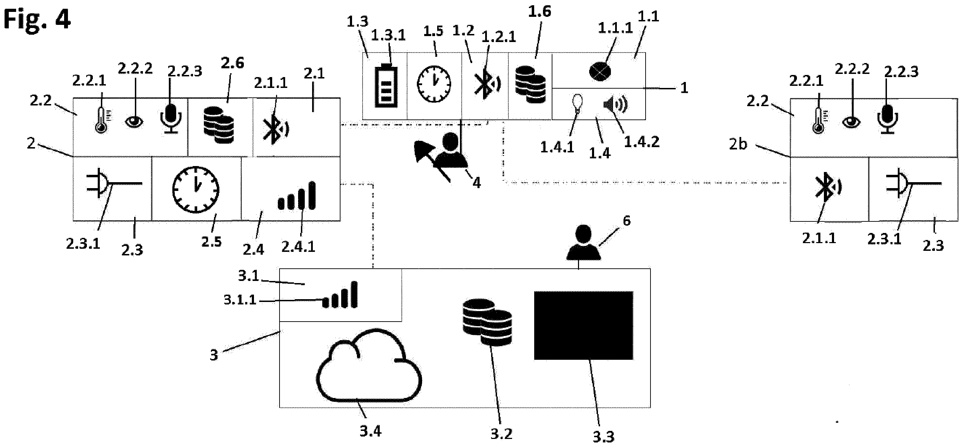

Figur 4 zeigt ein System zum Überwachen einer Arbeitssituation, welches wie das in Figur 1 gezeigte System eine mobile Sensoreinheit 1, eine zentrale Auswerteeinheit 3 und einen Sensorknoten 2 aufweist, wobei der Sensorknoten 2 gleichzeitig ein Kommunikationsknoten ist.

Darüber hinaus weist das System noch einen weiteren Sensorknoten 2b auf, der nicht als Kommunikationsknoten ausgebildet ist. Der Sensorknoten 2b wird über das Stromnetz mit Strom versorgt und verfügt als Sensoren 2.2 über einen Temperatursensor 2.2.1, einen Helligkeitssensor 2.2.2 und einen Schallsensor 2.2.3. Der weitere Sensorknoten 2b verfügt außerdem über eine Bluetooth-Antenne 2.1.1, die als Sendeeinheit eingerichtet ist und durchgängig ein Signal aussendet, das die von ihren Sensoren 2.2 zum jeweiligen Zeitpunkt erfassten Messwerte, also die jeweils aktuellen Messwerte zweiter physikalischer Größen, enthält.

Wenn sich die mobile Sensoreinheit 1 innerhalb der Reichweite des weiteren Sensorknotens 2b befindet, empfängt die mobile Sensoreinheit 1 über ihre Bluetooth-Antenne 1.2.1 das Signal mit den Messwerten, die von dem weiteren Sensorknoten 2b ausgesandt werden. Neben den Messwerten können in den ausgesandten Signalen noch Informationen enthalten sein, die eine Identifikation des weiteren Knotens 2b ermöglichen.

Die von der mobilen Sensoreinheit 1 auf diese Weise empfangenen Messwerte werden in einem Speicher 1.6 der mobilen Sensoreinheit 1 zwischengespeichert. Zeitgleich werden von dem Sensor 1.1 der mobilen Sensoreinheit selbst Messwerten einer oder mehrerer erster physikalischer Größen ermittelt und ebenfalls zwischengespeichert. Das Abspeichern erfolgt paarweise zugeordnet, so dass Messwerte erster und zweiter Größen, die gleichzeitig erhoben wurden, einander zugeordnet werden können. Auf diese Weise generierte Messwertpaare sind also geeignet, die Arbeitssituation des Individuums 4, das die mobile Sensoreinheit trägt, zeitaufgelöst zu beschreiben. Die abgespeicherten Messwertpaare werden mit einem Zeitstempel versehen, damit nachvollziehbar ist, wann diese Arbeitssituation vorgelegen hat. Die mobile Sensoreinheit verfügt dafür über eine Uhr 1.5. Die möglichen Daten zur Identifikation des weiteren Knotens 2b können ebenfalls auf der mobilen Sensoreinheit zwischengespeichert werden, damit nachvollziehbar ist, in der Umgebung welches Sensorknotens die Messwerte eines konkreten Messwertpaares erhoben wurden.

Der ebenfalls im System vorhandene Sensorknoten 2, der gleichzeitig als Kommunikationsknoten ausgebildet ist, hat im Wesentlichen die gleiche Funktionalität wie der im Zusammenhang mit Figur 1 beschriebene Sensorknoten. Er ist obendrein aber auch dazu eingerichtet, die im Speicher 1.6 der mobilen Sensoreinheit 1 zwischengespeicherten Daten, also die Messwertpaare mit dem Zeitstempel, zu empfangen, wenn die mobile Sensoreinheit sich innerhalb der Reichweite seiner Sende- und Empfangseinheit 2.1 befindet. Der Sensorknoten 2 weist ebenfalls eine Uhr 2.5 auf. Es ist vorgesehen, dass diese Uhr 2.5 mit der Uhr 1.5 der mobilen Sensoreinheit 1 abgeglichen wird. Dabei kann die Uhr 1.5 der mobilen Sensoreinheit 1 mit der Uhr 2.5 des Sensorknotens 2 synchronisiert werden, wenn eine Kommunikationsverbindung besteht. Wird nach einem Trennen und einem späteren erneuten Herstellen der Kommunikationsverbindung festgestellt, dass die Uhr 1.5 der mobilen Sensoreinheit 1 in der Zwischenzeit von der Uhr des Sensorknotens 2 abgewichen ist, so können die Zeitstempel der auf der mobilen Sensoreinheit 1 zwischengespeicherten Messwertpaare korrigiert werden. Die von dem Sensor- und Kommunikationsknoten 2 empfangenen Messwerte werden an die zentrale Auswerteeinheit übermittelt. Ebenso werden die auf dem Sensor- und Kommunikationsknoten 2 erhobenen Messwerte zweiter physikalischer Größen, gepaart mit den Messwerten erster physikalischer Größen, die auf der mobilen Sensoreinheit 1 erhoben werden, während diese sich in seiner Umgebung befindet, an die zentrale Auswerteeinheit übermittelt.

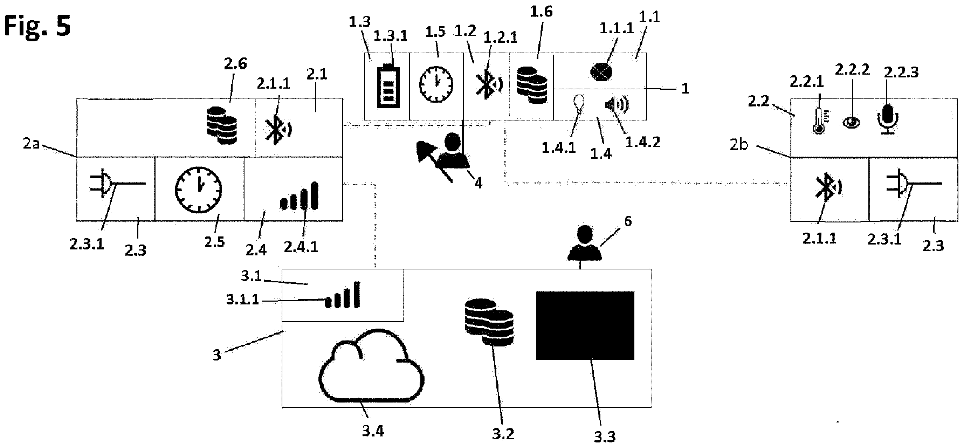

Figur 5 zeigt ein System, welches sich von dem System aus Figur 4 darin Unterscheidet, dass der Kommunikationsknoten 2a nicht gleichzeitig Sensorknoten ist. Der Kommunikationsknoten 2a ist also hier dazu da, die Messwertpaare, die zuvor im Speicher 1.6 der mobilen Sensoreinheit 1 zwischengespeichert waren zu empfangen und anschließend an die zentrale Auswerteeinheit 3 zu senden. Dieser Kommunikationsknoten 2a hat selbst keine Sensoren. Es ist wieder vorgesehen, dass der Kommunikationsknoten 2a und die mobile Sensoreinheit 1 für die im Zusammenhang mit Figur 4 beschriebene Synchronisation ihrer Uhren eingerichtet sind.

Der Kommunikationsknoten 2a, der kein Sensorknoten ist, kann beispielsweise in einem Meisterbüro oder einer Umkleidekabine bereitgestellt werden, wo keine Erhebung von Messwerten zweiter physikalischer Größen notwendig ist. Die mobilen Sensoreinheiten können dann in der Umgebung des Kommunikationsknotens 2a gelagert werden. Die einzelnen Individuen können dann vor ihrer Arbeit ihre mobile Sensoreinheit dort abholen und sie nach ihrer Arbeit wieder zurückbringen, damit der Datenaustausch zwischen der mobilen Sensoreinheit 1 und dem Kommunikationsknoten 2a stattfinden kann.

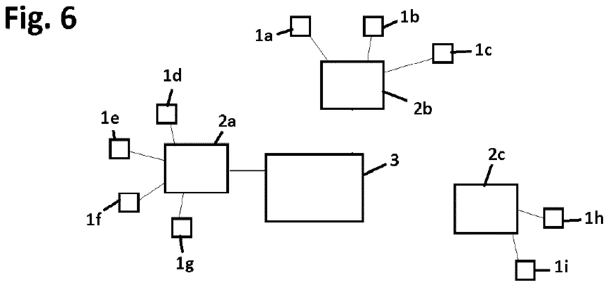

Figur 6 zeigt in der gleichen Darstellung wie Figur 3 ein typisches System, welches einen Kommunikationsknoten 2a und mehrere Sensorknoten 2b, 2c, die jeweils nicht als Kommunikationsknoten ausgebildet sind, umfasst. Gezeigt sind zwei Sensorknoten 2b, 2c, es können aber auch mehr als zwei sein. Nur der Kommunikationsknoten 2a ist zur Kommunikation mit der zentralen Auswerteeinheit 3 eingerichtet, wie in der Figur durch eine Linie gezeigt ist. Mit dem Kommunikationsknoten 2a und den Sensorknoten 2b, 2c kommunizieren die mobilen Sensoreinheiten 1a-1i jeweils immer dann, wenn sie sich in der Umgebung befinden. Sind die mobilen Sensoreinheiten 1 in der Umgebung eines Sensorknotens 2b, 2c, empfangen sie die auf den Sensorknoten 2b, 2c erhobenen Messwerte der zweiten physikalischen Größen und erheben zur gleichen Zeit selbst Messwerte erster physikalischer Größen. All diese Messwerte werden wie zuvor beschrieben auf den mobilen Sensoreinheiten 1 paarweise und zeitaufgelöst zwischengespeichert.

Werden die mobilen Sensoreinheiten 1 dann später in die Umgebung des Kommunikationsknotens 2a gebracht, werden die zwischengespeicherten Messwerte an den Kommunikationsknoten 2a und von dort an die zentrale Auswerteeinheit 3 weitergeleitet. Dabei kann es auch mehrere Kommunikationsknoten in dem System geben. Einige oder alle davon können gleichzeitig sensorknoten sein.

### Bezugszeichenliste

- 1, 1a-i: Mobile Sensoreinheit
- 1.1: Sensor
- 1.1.1: Bewegungssensor
- 1.2: Sender oder Sende- und Empfangseinheit
- 1.2.1: Bluetooth-Antenne
- 1.3: Energiespeicher
- 1.3.1: Akku
- 1.4: Feedback-Vorrichtung
- 1.5: Uhr
- 1.6: Speicher
- 1.4.1: Feedback-Lämpchen
- 1.4.2: Feedback-Lautsprecher
- 1.4.3: Feedback-Display
- 2: Sensor- und Kommunikationsknoten
- 2a: Kommunikationsknoten
- 2b-c: Sensorknoten
- 2.1: Empfänger oder Sende- und Empfangseinheit
- 2.1.1: Bluetooth-Antenne
- 2.2: Sensor
- 2.2.1: Temperatursensor
- 2.2.2: Helligkeitssensor
- 2.2.3: Schallsensor
- 2.3: Energieversorgung
- 2.3.1: Netzstecker
- 2.4: Kommunikationseinheit
- 2.4.1: Mobilfunkantenne
- 2.5: Uhr
- 2.6: Speicher
- 3: Zentrale Auswerteeinheit
- 3.1: Kommunikationseinheit
- 3.1.1: Mobilfunkantenne
- 3.2: Datenbank
- 3.3: Bedienerschnittstelle
- 3.4: Cloud
- 4: Individuum
- 5.1: Als Armband ausgebildetes Wearable
- 5.2: Als Handschuh ausgebildetes Wearable
- 5.3: Als Kleidungsstück ausgebildetes Wearable
- 6: Bediener

## Patentansprüche

1. System zum Überwachen einer Arbeitssituation, umfassend mindestens eine mobile Sensoreinheit (1, 1a-i), mindestens einen Kommunikationsknoten (2a), mindestens einen durch den Kommunikationsknoten (2a) gebildeten oder zusätzlich dazu vorgesehenen Sensorknoten (2, 2b, 2c) und eine zentrale Auswerteeinheit (3),
wobei die mindestens eine mobile Sensoreinheit (1, 1a-i) mindestens einen Sensor (1.1) aufweist, der zum Messen mindestens einer ersten physikalischen Größe eingerichtet ist, die zum Beschreiben einer Tätigkeit oder eines Zustands eines die mobile Sensoreinheit (1, 1a-i) tragenden Individuums (4) geeignet ist, und wobei der Sensor der mindestens einen mobilen Sensoreinheit (1, 1a-i) einen Beschleunigungssensor, ein Magnetometer oder ein Gyroskop ist oder einen Beschleunigungssensor und/oder ein Magnetometer und/oder ein Gyroskop umfasst,
wobei die mindestens eine mobile Sensoreinheit (1, 1a-i) einen Sender (1.2) zum drahtlosen Übertragen von durch den mindestens einen Sensor (1.1) dieser mobilen Sensoreinheit (1, 1a-i) erfassten Messwerten der mindestens einen ersten physikalischen Größe an den mindestens einen Kommunikationsknoten (2a) und einen Energiespeicher (1.3) zur Stromversorgung des Senders aufweist,
wobei der mindestens eine Sensorknoten (2, 2b, 2c) mindestens einen Sensor (2.2) aufweist, der zum Messen mindestens einer zweiten physikalischen Größe eingerichtet ist, die zum Beschreiben eines Zustands einer Umgebung des Sensorknotens (2, 2b, 2c) geeignet ist und der ein Temperatursensor oder ein Helligkeitssensor oder ein Vibrationssensor oder ein Schallsensor ist oder einen Temperatursensor und/oder einen Helligkeitssensor und/oder einen Vibrationssensor und/oder einen Schallsensor umfasst,
wobei der mindestens eine Kommunikationsknoten (2a) einen Empfänger (2.1) zum Empfangen der durch den Sender (1.2) der mobilen Sensoreinheit (1, 1a-i) übertragenen Messwerte der mindestens einen ersten physikalischen Größe und eine Kommunikationseinheit (2.4) zum Übertragen der durch den Empfänger (2.1) dieses Kommunikationsknotens (2a) empfangenen Messwerte der mindestens einen ersten physikalischen Größe und von durch den mindestens einen Sensor (2.2) des Sensorknotens (2, 2b, 2c) erfassten Messwerten der mindestens einen zweiten physikalischen Größe an die zentrale Auswerteeinheit (3) aufweist, und
wobei die zentrale Auswerteeinheit (3) eine Kommunikationseinheit (3.1) zum Empfangen der durch die Kommunikationseinheit (2.4) des Kommunikationsknotens (2a) übertragenen Messwerte der mindestens einen ersten physikalischen Größe und der mindestens einen zweiten physikalischen Größe aufweist,
wobei das System eingerichtet ist, die durch den mindestens einen Sensor (2.2) des Sensorknotens (2, 2b, 2c) erfassten Messwerte der mindestens einen zweiten physikalischen Größe der zumindest zu einem Zeitpunkt in der Umgebung dieses Sensorknotens (2, 2a-c) lokalisierten mobilen Sensoreinheit (1, 1a-i) zuzuordnen und zusammen mit den durch den mindestens einen Sensor (1.1) der zumindest zu dem Zeitpunkt in der Umgebung dieses Sensorknotens (2, 2a-c) lokalisierten mobilen Sensoreinheit (1, 1a-i) erfassten Messwerten der mindestens einen ersten physikalischen Größe als einander zugeordnet an die zentrale Auswerteeinheit (3) zu übermitteln und dort zu speichern und/oder weiterzuverarbeiten.

2. System nach Anspruch 1, wobei die mindestens eine mobile Sensoreinheit (1, 1a-i) eine Empfangseinheit aufweist und der mindestens eine Sensorknoten (2, 2b, 2c) eine Sendeeinheit aufweist, um eine bidirektionale drahtlose Kommunikation, vorzugsweise eine bidirektionale Bluetooth-Kommunikation, zwischen der mindestens einen mobilen Sensoreinheit (1, 1a-i) und dem mindestens einen Sensorknoten (2, 2b, 2c) zu ermöglichen und/oder eine Verwendung des Sensorknotens (2, 2b, 2c) als Funkbake zu ermöglichen.

3. System nach Anspruch 2, wobei die mindestens eine mobile Sensoreinheit (1, 1a-i) eingerichtet ist, durch mit ihrer Empfangseinheit empfangene Signale des als Funkbake wirkenden Sensorknotens (2, 2b, 2c) zu detektieren, wenn sie in der Umgebung dieses Sensorknotens (2, 2b, 2c) lokalisiert ist.

4. System nach einem der Ansprüche 2 oder 3, wobei die mindestens eine mobile Sensoreinheit (1, 1a-i) eingerichtet ist, die durch den mindestens einen Sensor (2.2) des mindestens einen Sensorknotens (2, 2b, 2c) erfassten Messwerte der mindestens einen zweiten physikalischen Größe mit ihrer Empfangseinheit zu empfangen, wenn sie in der Umgebung dieses Sensorknotens (2, 2b, 2c) lokalisiert ist, und mit ihrem Sender (1.2) zusammen mit den durch ihren mindestens einen Sensor (1.1) erfassten Messwerten der mindestens einen ersten physikalischen Größe an den Empfänger (2.1) des Kommunikationsknotens (2a) zu übertragen, wenn oder sobald der Kommunikationsknoten (2a) in Reichweite ihres Senders (1.2) ist.

5. System nach Anspruch 4, wobei die mindestens eine mobile Sensoreinheit (1, 1a-i) einen Speicher (1.6) zum Zwischenspeichern der durch den mindestens einen Sensor (2.2) des mindestens einen Sensorknotens (2, 2b, 2c) erfassten und mit ihrer Empfangseinheit empfangenen Messwerte der mindestens einen zweiten physikalischen Größe und der durch ihren mindestens einen Sensor (1.1) erfassten Messwerte der mindestens einen ersten physikalischen Größe aufweist und/oder eingerichtet ist, die durch den mindestens einen Sensor (2.2) des mindestens einen Sensorknotens (2, 2b, 2c) erfassten und mit ihrer Empfangseinheit empfangenen Messwerte der mindestens einen zweiten physikalischen Größe den durch ihren mindestens einen Sensor (1.1) erfassten zeitabhängigen Messwerten der mindestens einen ersten physikalischen Größe zeitlich zuzuordnen.

6. System nach Anspruch 5, wobei die mobile Sensoreinheit (1, 1a-i) und der Kommunikationsknoten (2a) jeweils eine Uhr (1.5, 2.5) aufweisen und eingerichtet sind, diese Uhren (1.5, 2.5) zu synchronisieren.

7. System nach einem der vorhergehenden Ansprüche, wobei die Kommunikationseinheit (2.4) des mindestens einen Kommunikationsknotens (2a) und die Kommunikationseinheit (3.1) der zentralen Auswerteeinheit (3) zur bidirektionalen Kommunikation zwischen dem mindestens einen Kommunikationsknoten (2a) und der zentralen Auswerteeinheit (3) ausgebildet sind.

8. System nach einem der vorhergehenden Ansprüche, wobei die Kommunikationseinheit (2.4) des mindestens einen Kommunikationsknotens (2a) und die Kommunikationseinheit (3.1) der zentralen Auswerteeinheit (3) als drahtlose Kommunikationseinheiten ausgebildet sind, vorzugsweise unter Verwendung von Mobilfunktechnologie, LAN oder WLAN.

9. System nach einem der vorhergehenden Ansprüche, wobei der Empfänger (2.1) des mindestens einen Kommunikationsknotens (2a) zum Lokalisieren der mobilen Sensoreinheit (1, 1a-i) eingerichtet ist.

10. System nach Anspruch 9, wobei der mindestens eine Kommunikationsknoten (2a) und die zentrale Auswerteeinheit (3) eingerichtet sind, die durch den mindestens einen Sensor (2.2) des durch diesen Kommunikationsknoten (2a) gebildeten Sensorknotens erfassten Messwerte der mindestens einen zweiten physikalischen Größe der durch den Empfänger (2.1) dieses Kommunikationsknotens (2a) in der Umgebung dieses Kommunikationsknotens (2a) lokalisierten mobilen Sensoreinheit (1, 1a-i) zuzuordnen und zusammen mit den durch den mindestens einen Sensor (1.1) der in der Umgebung dieses Kommunikationsknotens (2a) lokalisierten mobilen Sensoreinheit (1, 1a-i) erfassten Messwerten der mindestens einen ersten physikalischen Größe als einander zugeordnet zu speichern und/oder weiterzuverarbeiten.

11. System nach einem der vorhergehenden Ansprüche, wobei die mindestens eine mobile Sensoreinheit (1, 1a-i) als Wearable (5.1, 5.2, 5.3) ausgestaltet ist und/oder eine Feedback-Vorrichtung (1.4) zum Senden einer Information an das die mobile Sensoreinheit (1, 1a-i) tragende Individuum (4) umfasst.

12. Verfahren zum Überwachen einer Arbeitssituation unter Verwendung eines Systems nach einem der Ansprüche 1 bis 11 an einer Arbeitsstätte, vorzugsweise in Produktion, Logistik oder in einem Krankenhaus, wobei die mindestens eine mobile Sensoreinheit (1, 1a-i) von einem Individuum (4) oder von jeweils einem mehrerer Individuen (4) beim Ausüben von Tätigkeiten an der Arbeitsstätte getragen wird, wobei der mindestens eine Sensorknoten (2, 2b, 2c) an einem Ort oder mehreren Orten der Arbeitsstätte angeordnet ist und der Empfänger des mindestens einen Kommunikationsknotens die mobile Sensoreinheit lokalisiert.

13. Verfahren nach Anspruch 12,
- wobei dem Individuum (4) oder mindestens einem der Individuen (4) abhängig von den diesem Individuum (4) zugeordneten Messwerten der mindestens einen ersten und der mindestens einen zweiten physikalischen Größe eine Rückmeldung gegeben wird zur Verbesserung einer Ergonomie der von dem Individuum (4) oder den Individuen (4) ausgeübten Tätigkeiten, vorzugsweise zur Reduktion physischer und/oder psychischer Belastungen des Individuums oder der Individuen (4) oder
- wobei die Messwerte der mindestens einen ersten physikalischen Größe so gespeichert werden, dass keine Zuordnung dieser Messwerte zu der mobilen Sensoreinheit (1, 1a-i), mit deren mindestens einem Sensor (1.1) sie erfasst worden sind, und/oder zu dem Individuum (4), das diese mobile Sensoreinheit (1, 1a-i) trägt oder getragen hat, mehr möglich ist.

## Claims

1. System for monitoring a working situation, comprising at least one mobile sensor unit (1, 1a-i), at least one communication node (2a), at least one sensor node (2, 2b, 2c) which is formed by the communication node (2a) or is provided in addition thereto, and a central evaluation unit (3),
wherein the at least one mobile sensor unit (1, 1a-i) comprises at least one sensor (1.1) configured for measuring at least one first physical variable suitable for describing an activity or a state of an individual (4) carrying the mobile sensor unit (1, 1a-i), and wherein the sensor of the at least one mobile sensor unit (1, 1a-i) is an acceleration sensor, a magnetometer or a gyroscope or comprises an acceleration sensor and/or a magnetometer and/or a gyroscope,
wherein the at least one mobile sensor unit (1, 1a-i) comprises a transmitter (1.2) for a wireless transmission of measured values of the at least one first physical variable, which are detected by the at least one sensor (1.1) of this mobile sensor unit (1, 1a-i), to the at least one communication node (2a), and an energy storage means (1.3) for supplying power to the transmitter,
wherein the at least one sensor node (2, 2b, 2c) comprises at least one sensor (2.2), which is configured for measuring at least one second physical variable suitable for describing a state of an environment of the sensor node (2, 2b, 2c) and is a temperature sensor or a brightness sensor or a vibration sensor or a sound sensor or comprises a temperature sensor and/or a brightness sensor and/or a vibration sensor and/or a sound sensor,
wherein the at least one communication node (2a) comprises a receiver (2.1) for receiving the measured values of the at least one first physical variable, which are transmitted by the transmitter (1.2) of the mobile sensor unit (1, 1a-i), and a communication unit (2.4) for transmitting the measured values of the at least one first physical variable, which are received by the receiver (2.1) of this communication node (2a), and measured values of the at least one second physical variable, which are detected by the at least one sensor (2.2) of the sensor node (2, 2b, 2c), to the central evaluation unit (3), and
wherein the central evaluation unit (3) comprises a communication unit (3.1) for receiving the measured values of the at least one first physical variable and of the at least one second physical variable, which are transmitted by the communication unit (2.4) of the communication node (2a),
wherein the system is configured to assign the measured values of the at least one second physical variable, which are detected by the at least one sensor (2.2) of the sensor node (2, 2b, 2c), to the mobile sensor unit (1, 1a-i), which is located in the environment of this sensor node (2, 2a-c) at least at one point in time, and to transfer these together with the measured values of the at least one first physical variable, which are detected by the at least one sensor (1.1) of the mobile sensor unit (1, 1-i), which is located in the environment of this sensor node (2, 2a-c) at least at said point in time, to the central evaluation unit (3) as being assigned to one another and to store said measured values there and/or process them further.

2. System according to claim 1, wherein the at least one mobile sensor unit (1, 1a-i) comprises a receiving unit and the at least one sensor node (2, 2b, 2c) comprises a transmitting unit, in order to permit bidirectional wireless communication, preferably bidirectional Bluetooth communication, between the at least one mobile sensor unit (1, 1a-i) and the at least one sensor node (2, 2b, 2c) and/or to permit use of the sensor node (2, 2b, 2c) as a radio beacon.

3. System according to claim 2, wherein the at least one mobile sensor unit (1, 1a-i) is configured, by way of signals from the sensor node (2, 2b, 2c) received by its receiving unit, said sensor node acting as a radio beacon, to detect if it is located in the environment of this sensor node (2, 2b, 2c).

4. System according to either of claims 2 or 3, wherein the at least one mobile sensor unit (1, 1a-i) is configured to receive the measured values of the at least one second physical variable, which are detected by the at least one sensor (2.2) of the at least one sensor node (2, 2b, 2c), by means of its receiving unit, when it is located in the environment of this sensor node (2, 2b, 2c), and using its transmitter (1.2) to transmit said values together with the measured values of the at least one first physical variable, which are detected by its at least one sensor (1.1), to the receiver (2.1) of the communication node (2a) when or as soon as the communication node (2a) is within range of its transmitter (1.2).

5. System according to claim 4, wherein the at least one mobile sensor unit (1, 1a-i) comprises a memory (1.6) for intermediately storing the measured values of the at least one second physical variable, which are detected by the at least one sensor (2.2) of the at least one sensor node (2, 2b, 2c) and which are received by its receiving unit, and the measured values of the at least one first physical variable, which are detected by its at least one sensor (1.1), and/or is configured to temporally assign the measured values of the at least one second physical variable, which are detected by the at least one sensor (2.2) of the at least one sensor node (2, 2b, 2c) and are received by its receiving unit, to the time-dependent measured values of the at least one first physical variable, which are detected by its at least one sensor (1.1).

6. System according to claim 5, wherein the mobile sensor unit (1, 1a-i) and the communication node (2a) each comprise a clock (1.5, 2.5) and are configured to synchronise these clocks (1.5, 2.5).

7. System according to any of the preceding claims, wherein the communication unit (2.4) of the at least one communication node (2a) and the communication unit (3.1) of the central evaluation unit (3) are configured for the bidirectional communication between the at least one communication node (2a) and the central evaluation unit (3).

8. System according to any of the preceding claims, wherein the communication unit (2.4) of the at least one communication node (2a) and the communication unit (3.1) of the central evaluation unit (3) are configured as wireless communication units, preferably using mobile radio technology, LAN or WLAN.

9. System according to any of the preceding claims, wherein the receiver (2.1) of the at least one communication node (2a) is configured for locating the mobile sensor unit (1, 1a-i).

10. System according to claim 9, wherein the at least one communication node (2a) and the central evaluation unit (3) are configured to assign the measured values of the at least one second physical variable, which are detected by the at least one sensor (2.2) of the sensor node formed by this communication node (2a), to the mobile sensor unit (1, 1a-i) located in the environment of this communication node (2a) by the receiver (2.1) of this communication node (2a) and to store said values as being assigned to one another and/or further process them together with the measured values of the at least one first physical variable, which are detected by the at least one sensor (1.1) of the mobile sensor unit (1, 1a-i), which is located in the environment of this communication node (2a).

11. System according to any of the preceding claims, wherein the at least one mobile sensor unit (1, 1a-i) is configured as a wearable (5.1, 5.2, 5.3) and/or comprises a feedback device (1.4) for sending information to the individual (4) carrying the mobile sensor unit (1, 1a-i).

12. Method for monitoring a working situation using a system according to any of claims 1 to 11 at a working site, preferably in production, logistics or in a hospital, wherein the at least one mobile sensor unit (1, 1a-i) is carried by an individual (4) or by each one of several individuals (4) while carrying out activities at the working site, wherein the at least one sensor node (2, 2b, 2c) is arranged at one location or several locations of the working site or sites and the receiver of the at least one communication node locates the mobile sensor unit.

13. Method according to claim 12,
- wherein feedback is provided to the individual (4) or at least one of the individuals (4) depending on the measured values of the at least one first and the at least one second physical variable, which are assigned to this individual (4), in order to improve ergonomics of the activities carried out by the individual (4) or individuals (4), preferably to reduce physical and/or psychological stresses on the individual or individuals (4), or
- wherein the measured values of the at least one first physical variable are stored such that it is no longer possible to assign these measured values to the mobile sensor unit (1, 1a-i), which values have been detected by means of its at least one sensor (1.1), and/or to the individual (4) who is carrying or has carried this mobile sensor unit (1, 1a-i).

## Revendications

1. Système de surveillance d'une situation de travail, comprenant au moins une unité de capteur mobile (1, 1a-i), au moins un nœud de communication (2a), au moins un nœud de capteur (2, 2b, 2c) formé par le nœud de communication (2a) ou prévu en plus de celui-ci et une unité d'exploitation centrale (3),
dans lequel l'au moins une unité de capteur mobile (1, 1a-i) présente au moins un capteur (1.1) qui est conçu pour la mesure d'au moins une première grandeur physique, qui est appropriée pour décrire une tâche ou un état d'un individu (4) transportant l'unité de capteur mobile (1, 1a-i), et dans lequel le capteur de l'au moins une unité de capteur mobile (1, 1a-i) est un capteur d'accélération, un magnétomètre ou un gyroscope, ou comprend un capteur d'accélération et/ou un magnétomètre et/ou un gyroscope,
dans lequel l'au moins une unité de capteur mobile (1, 1a-i) présente un émetteur (1.2) pour la transmission sans fil de valeurs de mesure de l'au moins une première grandeur physique détectées par l'au moins un capteur (1.1) de cette unité de capteur mobile (1, 1a-i) à l'au moins un nœud de communication (2a) et un accumulateur d'énergie (1.3) pour l'alimentation en courant de l'émetteur,
dans lequel l'au moins un nœud de capteur (2, 2b, 2c) présente au moins un capteur (2.2), qui est conçu pour la mesure d'au moins une deuxième grandeur physique qui est appropriée pour la description d'un état d'un environnement du nœud de capteur (2, 2b, 2c) et qui est un capteur de température ou un capteur de luminosité ou un capteur de vibrations ou un capteur de bruits ou comprend un capteur de température et/ou un capteur de luminosité et/ou un capteur de vibrations et/ou un capteur de bruits,
dans lequel l'au moins un nœud de communication (2a) présente un récepteur (2.1) pour la réception des valeurs de mesure de l'au moins une première grandeur physique transmises par l'émetteur (1.2) de l'unité de capteur mobile (1, 1a-i) et une unité de communication (2.4) pour la transmission des valeurs de mesure de l'au moins une première grandeur physique reçues par le récepteur (2.1) de ce nœud de communication (2a) et de valeurs de mesure de l'au moins une deuxième grandeur physique détectées par l'au moins un capteur (2.2) du nœud de capteur (2, 2b, 2cc) à l'unité d'exploitation centrale (3), et
dans lequel l'unité d'exploitation centrale (3) présente une unité de communication (3.1) pour la réception des valeurs de mesure de l'au moins une première grandeur physique et de l'au moins une deuxième grandeur physique transmises par l'unité de communication (2.4) du nœud de communication (2a),
où le système est conçu pour attribuer les valeurs de mesure de l'au moins une deuxième grandeur physique détectées par l'au moins un capteur (2.2) du nœud de capteur (2, 2b, 2c) à l'unité de capteur mobile (1, 1a-i) localisée à ce moment dans l'environnement de ce nœud de capteur (2, 2a-c) et pour les transmettre comme attribué les unes aux autres à l'unité d'exploitation centrale (3) conjointement avec les valeurs de mesure de l'au moins une première grandeur physique détectées par l'au moins un capteur(1.1) de l'unité de capteur mobile (1, 1a-i) localisée à ce moment dans l'environnement de ce nœud de capteurs (2, 2a-c) et pour les y stocker et/ou traiter ultérieurement.

2. Système selon la revendication 1, dans lequel l'au moins une unité de capteur mobile (1, 1a-i) présente une unité de réception et l'au moins un nœud de capteur (2, 2b, 2c) présente une unité de transmission, afin de permettre une communication bidirectionnelle sans fil, de préférence, une communication bidirectionnelle Bluetooth, entre l'au moins une unité de capteur mobile (1, 1a-i) et l'au moins un nœud de capteur (2, 2b, 2c) et/ou permettre un emploi du nœud de capteur (2, 2b, 2c) en tant que balise radio.

3. Système selon la revendication 2, dans lequel l'au moins une unité de capteur mobile (1, 1a-i) est conçue pour détecter des signaux du nœud de capteur (2, 2b, 2c) agissant en tant que balise radio reçus par son unité de réception lorsqu'elle est localisée dans l'environnement de ce nœud de capteur (2, 2b, 2c).

4. Système selon l'une des revendications 2 ou 3, dans lequel l'au moins une unité de capteur mobile (1, 1a-i) est conçue pour recevoir les valeurs de mesure de l'au moins une deuxième grandeur physique détectées par l'au moins un capteur (2.2) de l'au moins un nœud de capteur (2, 2b, 2c) avec son unité de réception, lorsqu'elle est localisée dans l'environnement de ce nœud de capteur (2, 2b, 2c), et les transmettre avec son émetteur (1.2) conjointement avec les valeurs de mesure de l'au moins une première grandeur physique détectées par l'au moins un capteur (1.1) au récepteur (2.1) du nœud de communication (2a) lorsque ou dès que le nœud de communication (2a) est à portée de son émetteur (1.2).

5. Système selon la revendication 4, dans lequel l'au moins une unité de capteur mobile (1, 1a-i) présente une mémoire (1.6) permettant de stocker provisoirement les valeurs de mesure de l'au moins une deuxième grandeur physique détectées par l'au moins un capteur (2.2) de l'au moins un nœud de capteurs (2, 2b, 2c) et reçues par son unité de réception et des valeurs de mesure de l'au moins une première grandeur de mesure détectées par l'au moins un capteur (1.1), et/ou est conçue pour classer dans le temps les valeurs de mesure de l'au moins une deuxième grandeur physique détectées par l'au moins un capteur (2.2) de l'au moins un nœud de capteur (2, 2b, 2c) et les valeurs de mesure de l'au moins une deuxième grandeur physique reçues avec son unité de réception et les valeurs de mesure de l'au moins une première grandeur physique dépendantes du temps détectées par son au moins un capteur (1.1).

6. Système selon la revendication 5, dans lequel l'unité de capteur mobile (1, 1a-i) et le nœud de communication (2a) présentent respectivement une horloge (1.5, 2.5) et sont conçus pour synchroniser ces horloges (1.5,2.5).

7. Système selon l'une des revendications précédentes, dans lequel l'unité de communication (2.4) de l'au moins un nœud de communication (2a) et l'unité de communication (3.1) de l'unité d'exploitation centrale (3) sont conçues pour la communication bidirectionnelle entre l'au moins un nœud de communication (2a) et l'unité d'exploitation centrale (3).

8. Système selon l'une des revendications précédentes, dans lequel l'unité de communication (2.4) de l'au moins un nœud de communication (2a) et l'unité de communication (3.1) de l'unité d'exploitation centrale (3) sont conçues sous forme d'unités de communication sans fil, de préférence moyennant l'emploi de la technologie radio mobile, LAN ou WLAN.

9. Système selon l'une des revendications précédentes, dans lequel le récepteur (2.1) de l'au moins un nœud de communication (2a) est conçu pour la localisation de l'unité de capteur mobile (1, 1a-i).

10. Système selon la revendication 9, dans lequel l'au moins un nœud de communication (2a) et l'unité d'exploitation centrale (3) sont conçus pour classer les valeurs de mesure de l'au moins une deuxième grandeur physique détectées par l'au moins un capteur (2.2) du nœud de capteur formé par ce nœud de communication (2a) de l'unité de capteurs mobile (1, 1a-i) localisée par le récepteur (2.1) de ce nœud de communication (2a) dans l'environnement de ce nœud de communication (2a) et pour stocker à l'état classées les unes par rapport aux autres conjointement avec les valeurs de mesure de l'au moins une première grandeur physique détectées par l'au moins un capteur (1.1) de l'unité de capteur mobile (1, 1a-i) localisée dans l'environnement de ce nœud de communication (2a), et/ou de les traiter ultérieurement.

11. Système selon l'une des revendications précédentes, dans lequel l'au moins une unité de capteur mobile (1, 1a-i) est conçue comme un dispositif « Wearable » (5.1, 5.2, 5.3) et/ou comprend un dispositif « Feedback » (1.4) pour la transmission d'une information à l'individu (4) transportant l'unité de capteur mobile (1, 1a-i).

12. Procédé de surveillance d'une situation de travail moyennant l'emploi d'un système selon l'une des revendications 1 à 11 au niveau d'un lieu de travail, de préférence dans la production, la logistique ou dans un hôpital, dans lequel l'au moins une unité de capteur mobile (1, 1a-i) est transportée par un individu (4), ou respectivement par un parmi plusieurs individus (4), lors de l'exercice de tâches sur le lieu de travail, dans lequel l'au moins un nœud de capteur (2, 2b, 2c) est disposé à un endroit ou à plusieurs endroits du lieu de travail et le récepteur de l'au moins un nœud de communication localise l'unité de capteur mobile.

13. Procédé selon la revendication 12,
- dans lequel une information de retour est donnée à l'individu (4), ou à au moins un des individus (4), en fonction de valeurs de mesure de l'au moins une première et de l'au moins une deuxième grandeur de mesure classées par cet individu (4) pour l'amélioration d'une ergonomie des tâches exercées par l'individu (4), ou les individus (4), de préférence pour la réduction de charges physiques et/ou psychiques de l'individu ou des individus (4), ou
- dans lequel les valeurs de mesure de l'au moins une première grandeur physique sont stockées de telle manière qu'aucune corrélation de ces valeurs de mesure n'est plus possible par rapport à l'unité de capteur mobile (1, 1a-i), avec l'au moins un capteur (1.1) par lequel elles ont été détectées, et/ou par rapport à l'individu (4), qui transporte ou a transporté cette unité de capteur mobile (1, 1a-i).
